# EUROPÄISCHE PATENTSCHRIFT

(11) **EP 0 973 583 B1**
(45) Veröffentlichungstag und Bekanntmachung des Hinweises auf die Patenterteilung: **07.04.2004**
(21) Anmeldenummer: 99907247.3
(22) Anmeldetag: 18.01.1999
(51) Int. Cl.: A61N 5/06

(54) **APPLIKATIONSVORRICHTUNG FÜR DIE BEHANDLUNG BIOLOGISCHER GEWEBE MIT LASERSTRAHLUNG**
APPLICATION DEVICE FOR TREATING BIOLOGICAL TISSUES WITH LASER RADIATION
DISPOSITIF D'APPLICATION PERMETTANT DE TRAITER DES TISSUS BIOLOGIQUES PAR UN RAYONNEMENT LASER

(30) Priorität: 30.01.1998 DE 19803460
(43) Veröffentlichungstag der Anmeldung: 26.01.2000
(73) Patentinhaber: Dornier Medtech GmbH, 82234 Wessling (DE)
(72) Erfinder: ROTHER, Werner, D-64331 Weiterstadt (DE); HAUPTMANN, Gerhard, D-81827 München (DE)
(74) Vertreter: Grünecker, Kinkeldey, Stockmair & Schwanhäusser Anwaltssozietät
(86) Internationale Anmeldenummer: PCT/DE1999/000102
(87) Internationale Veröffentlichungsnummer: WO 1999/038571

(56) Entgegenhaltungen:
- DE-A- 4 137 983
- DE-A- 4 403 134
- FR-A- 2 714 147
- US-A- 4 732 442
- US-A- 5 151 096
- PATENT ABSTRACTS OF JAPAN vol. 7, no. 78 (P-188), 31. März 1983 & JP 58 007604 A (MITSUBUSHI DENKI KK), 17. Januar 1983

## Beschreibung

Die Erfindung betrifft eine Applikationsvorrichtung für die Behandlung biologischer Gewebe mit Laserstrahlung, mit einem die Laserstrahlung führenden Lichtleiter, dessen distales Ende zumindest teilweise von der Mantelschicht (cladding) befreit ist, derart, daß die Laserstrahlung seitlich aus dem Faserkern austritt.

In der US 5151096 ist eine Vorrichtung der o. g. Art beschrieben, bei welcher das Laserlicht entlang des vom Mantel befreiten distalen Bereiches seitlich austritt. Um den dort auftretenden exponentialen Abfall der abgestrahlten Laserlichtleistung etwas zu kompensieren wird dort das aus dem distalen Ende des Lichtleiters austretende Licht reflektiert und in Richtung des proximalen Ende des Lichtleiters abgestrahlt.. Die Intensität der abgestrahlten Laserlichtleistung nimmt hier in Richtung distalem Ende des Lichtleiters zunächst ab und steigt dann im Endbereich des Lichtleiters wieder etwas an. Trotz des hohen Aufwandes ergibt sich dennoch keine homogene Intensitätsverteilung. Aus dieser Schrift ist es zudem bekannt, zumindest die von der Mantelschicht befreiten Stellen des Lichtleiters mit einem für die Laserstrahlung durchlässigen Koppelmedium versehen, welches den gleichen oder zumindest einen ähnlichen Brechungsindex aufweist wie der Faserkern, wodurch erreicht wird, daß das Licht seitlich aus dem Faserkern austreten kann.

Aus der DE OS 4403134 A1 ist eine andere Methode bekannt, durch welche das seitliche Austreten des Laserlichts aus dem distalen Ende einer Lichtleitfaser erreicht werden soll. Von dort ist es bekannt die Mantelfläche des freigelegten Faserkerns entweder durch unterschiedliche Maßnahmen aufzurauhen oder mit Kerben oder Stufen zu versehen, an welchen das austretende Licht zusätzlich gestreut bzw. aus der ursprünglichen Strahlrichtung abgelenkt wird. Auf diese Weise wird zwar der Abstrahlungsbereich entlang des distalen Endes des Lichtleiters erreicht, diese Vorrichtung hat jedoch neben dem hohen Herstellungsaufwand ebenfalls den Nachteil, daß der Intensitätsverlauf der am distalen Ende abgestrahlten Laserlichtleistung inhomogen ausgebildet ist. Die Intensität der abgestrahlten Laserlichtleistung nimmt in Richtung distalem Ende des Lichtleiters exponentiell ab.

Vorrichtungen der o. g. Art sind beispielsweise. aus der DE 4137 983 A1 bekannt und werden vornehmlich für interstitielle Behandlungsarten, insbesondere für die interstitielle Thermotherapie (ITT) verwendet. Für die Behandlung von gutartigen Tumoren im urologischen Fachbereich ist die ITT zur Standardbehandlung geworden. Die Übertragung dieser Methode auf maligne Tumoren wird jedoch erst möglich sein, wenn pro Behandlung größere Schädigungsareale erreicht werden können. Bei bösartigen Tumoren muß im Gegensatz zu gutartigen Tumoren sichergestellt sein, daß der Tumorherd zu hundert Prozent zerstört wird. Aufgrund der im wesentlichen kugelförmigen Intensitätsverteilung der abgestrahlten Laserlichtleistung ist bei einem Einsatz der aus der DE 4137 983 A1 bekannten Vorrichtung nicht sichergestellt, daß der Tumorherd zu hundert Prozent zerstört wird.

Laserlicht im Wellenlängenbereich zwischen 800 und 1100 um dringt je nach Gewebetyp etwa 2 bis 5 mm tief in das Gewebe ein, bis es vollständig absorbiert ist. Bestrahlt man Gewebe mit dieser Wellenlänge, erwärmt es sich zunächst durch direkte Absorption der Laserstrahlung und anschließend nur noch durch Wärmeleitung. Eine Vergrößerung der Schädigungszone über die unmittelbare Eindringtiefe der Laserstrahlung hinaus kann nur durch Wärmeleitung, d.h. durch Verlängerung der Bestrahlungszeit oder durch Vergrößerung der abstrahlenden Fläche des Applikators bei sonst unveränderter Applikatorgeometrie erreicht werden.

Ein weiteres Anwendungsgebiet für die genannte Applikationsvorrichtung ist die photodynamische Therapie (PDT) bei welcher dem Patienten ein photosensibles Mittel, z.B. Photoporphyrine injiziert wird, welches sich vorwiegend im Tumorgewebe ablagert. Wird das Gewebe mit Licht einer entsprechenden Wellenlänge bestrahlt, entstehen Toxine als Folge von chemischen Reaktionen im photo sensiblen Mittel. Auf diese Weise wird das Tumorareal selektiv geschädigt.

Um nun alle Tumorbereiche gleichermaßen zu erreichen, ist eine möglichst homogene Lichtverteilung des Laserlichtes erforderlich.

Es ist daher Aufgabe der vorliegenden Erfindung eine Applikationsvorrichtung der o.g. Art zu schaffen, welche bei einfacher Herstellung eine möglichst gleichmäßige Lichtverteilung über einen großen Längenabschnitt des distalen Lichtleiterendes ermöglicht.

Diese Aufgabe wird durch eine Applikationsvorrichtung mit den Merkmalen des Patentanspruchs 1 gelöst.

Die Überlegungen, die zur Entstehung der vorliegenden Erfindung führten gingen davon aus, daß man der zum distalen Ende des Lichtleiters hin exponientiell abfallenden Intensitätsverteilung des abgestrahlten Lichtes dadurch entgegenwirken kann, indem man durch geeignete Maßnahmen verhindert, daß das Laserlicht hauptsächlich am Beginn des Abstrahlungsbereiches austritt und gleichzeitig das Austreten von Laserlicht dem Ende des Abstrahlungsbereiches hinzu erleichtert.

Das distale Ende im Bereich der von der Mantelschicht befreiten Stellen des Lichtleiters wird mit einem für die Laserstrahlung durchlässigen Koppelmedium versehen, welches den gleichen oder zumindest einen ähnlichen Brechungsindex aufweist wie der Faserkern, hierdurch wird erreicht, daß das Licht seitlich aus dem Faserkern austreten kann.

Da das Licht innerhalb eines Lichtleiters ständig an der Grenzschicht zwischen dem Faserkern und der umgebenden Mantelschicht, dem sog. Cladding reflektiert wird, tritt das Licht nur dort seitlich aus dem Faserkern aus, wo das cladding entfernt ist. An jeder vom cladding befreiten Stelle tritt Licht aus, wodurch sich die im Lichtleiter verbleibende Menge verringert. Um nun eine möglichst homogene Verteilung der ausgestrahlten Lichtmenge zu erreichen wird in Richtung distalem Ende immer mehr vom cladding entfernt, um so zu kompensieren, daß bereits an vorhergehenden Stellen Licht aus dem Lichtleiter ausgetreten ist und daher der Lichtleiter nicht mehr so viel Licht führt. Nach jeder vom cladding befreiten Stelle des Lichtleiters wird eine immer geringer Lichtmenge im Lichtleiter geführt. Daher muß der vom cladding befreite Bereich ständig vergrößert werden um auch dort die im wesentlichen gleiche Menge an Licht austreten zu lassen wie an den vorhergegangenen freigelegten Stellen.

Eine derartige Applikationsvorrichtung besitzt den Vorteil, daß an dem Lichtleiterkern nach der mechanisch einfachen Entfernung der Mantelschicht keinerlei mechanische oder chemische Ätz-, Schleif- oder Einkerbvorgänge erforderlich sind.

Die Erfindung wird im folgenden anhand der in den Figuren teilweise schematisch dargestellten Ausführungsbeispiele näher beschrieben.

Es zeigen:
- Fig. 1: einen Querschnitt durch das distale Ende einer Applikationsvorrichtung
und
- Fig 2a und b: Seitenansichten auf das distale Ende zweier unterschiedlich abgemantelter Lichtleiter

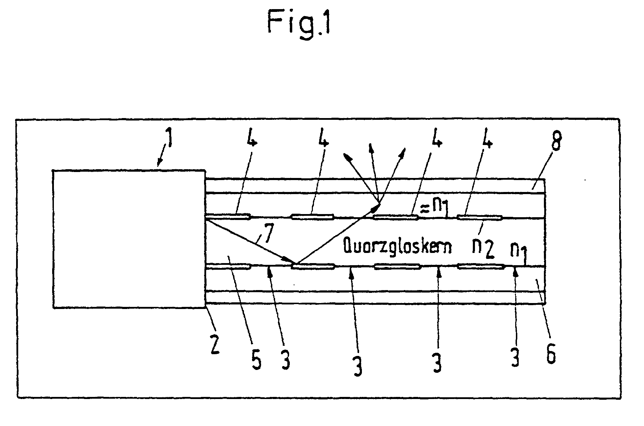

Bei dem in Fig. 1 dargestellten Ausführungsbeispiel ist das Ende eines Lichtleiters 1 sowohl von seiner, noch in Seitenansicht erkennbaren Umhüllung (coating) 2 als auch, in ringförmigen Abschnitten 3, von seiner Mantelschicht (cladding) 4 befreit. In diesen ringförmigen Abschnitten 3 liegt der Faserkem 5, welcher aus Quarzglas mit einem Mantelschicht 4 noch vorhanden ist, besteht der für die Führung von Licht notwendige Brechungsindex n₁ besteht, frei. In den ringförmigen Bereichen, bei denen die Brechungsindexsprung von n₁ auf den kleineren Brechungsindex n₂ der Mantelschicht 4.

Das derart durch Abschaben oder -Schälen präparierte distale Ende des Lichtleiters wird nun mit einem Koppelmedium 6 überzogen, welches ein Gemisch aus einem optisch transparenten aushärtbaren Kleber und einer lichtstreuenden Substanz, vorteilhafterweise ein Silikatkeramikpulver, ist. Der Kleber weist dabei einen Brechungsindex auf, der in etwa demjenigen des Faserkerns entspricht, also gleich oder ungefähr gleich n₁ ist.

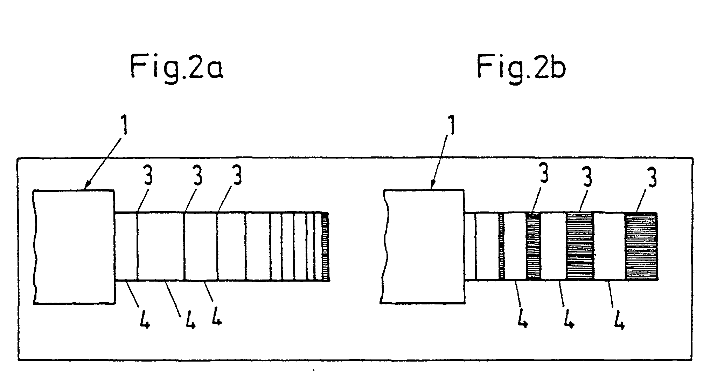

Ein im Faserkem geführter Lichtstrahl 7 wird folglich im Bereich des distalen Endes so lange im Faserkern weitergeführt, bis er auf einen von der Mantelschicht 4 befreiten Bereich trifft. Dort tritt er aus dem Faserkem 5 in das Koppelmedium 6 über, bis es darin auf ein Partikel der lichtstreuenden Substanz trifft, von welchem es dann nach bekannten physikalischen Gesetzen in denen die Partikelgröße einen wesentlichen Einfluß hat, gestreut wird. Die Intensität der jeweils im Lichtleiter verbleibenden Strahlung nimmt bei vollständig entmanteltem Faserkern mit der Länge des distalen, abgemantelten Endes in etwa exponentiell ab. Da jedoch eine an der Applikatoroberfläche homogene Lichtverteilung gewünscht wird, ist es erfindungsgemäß vorgesehen, die freigelegte Fläche des Faserkerns pro Längeneinheit in distaler Richtung ebenfalls exponentiell zu vergrößern. Dies kann gemäß Fig. 2a beispielsweise dadurch geschehen, daß die Abstände von jeweils gleich breiten Bereichen 3, in welchen die Mantelschicht 4 entfernt wurde, in distaler Richtung exponentiell verringert werden oder in dem gemäß Fig 2b die abgemantelten Flächen, bei gleichen Abständen, exponentiell größer werden. Um einen besseren mechanischen Schutz des Faserkerns zu gewährleisten, ist es vorteilhaft, das distale Ende in bekannterweise mit einer transparenten Hülse 8, z.B. aus Quarzglas, zu versehen.

Da bei üblicherweise prograder Einkopplung der Strahlung in den Lichtleiter bei einer gebräuchlichen Applikatorlänge nicht die gesamte Strahlung seitlich ausgekoppelt werden kann, würde ein nicht unerheblicher Teil aus der Stirnfläche austreten und dort zu ungewünschten Leistungsspitzen führen. Es hat sich nun gezeigt, daß der Einkoppelwinkel der Strahlung maßgebend für die Höhe der seitlich auskoppelbaren Energie ist. Damit nun alle Strahlungsmoden seitlich in das umgebende Material ausgekoppelt werden können, müssen die axialen Moden ausgeblendet werden. Eine derartige Ausblendung der axialen Moden geschieht in an sich bekannterweise durch schräge Strahleinkopplung in den Lichtleiter. Dazu kann entweder die fokussierte Laserstrahlung unter einem schrägen Winkel auf eine senkrecht zur Faserachse erzeugte Stirnfläche des Faserkerns eingekoppelt werden oder, der Lichtleiter wird an der proximalen Stirnfläche schräg angeschliffen und das Laserlicht in üblicherweise in prograder Richtung auf diese Stirnfläche fokussiert.

Insbesondere bei Verwendung von Diodenlasersystemen, bei welchen es schwierig ist axiale Moden durch Schrägeinkopplung auszublenden, empfiehlt sich eine Verformung der Lichtleiterspitze.

## Patentansprüche

1. Applikationsvorrichtung für die Behandlung biologischer Gewebe mit Laserstrahlung, mit einem die Laserstrahlung führenden Lichtleiter (1), dessen distales Ende zumindest teilweise von der Mantelschicht (4) befreit ist, derart, daß die Laserstrahlung seitlich aus dem Faserkem (5) austritt wobei zumindest die von der Mantelschicht (4) befreiten Stellen des distalen Lichtleiterendes mit einem für die Laserstrahlung durchlässigen Koppelmedium (6) versehen ist, welches einen gleichen oder ähnlichen Brechungsindex aufweist wie der Faserkem (5) und in welches eine lichtstreuende Substanz eingelagert ist,
**dadurch gekennzeichnet, daß**
die Mantelschicht (4) in mehreren ringförmigen Bereichen (3) entfernt ist, derart, daß die Mantelfläche des freigelegten Faserkems (5) pro Längeneinheit in distaler Richtung exponentiell zunimmt.

2. Applikationsvorrichtung nach Anspruch 1
**dadurch gekennzeichnet, daß**
das Koppelmedium (6) ein aushärtbarer Kleber ist.

3. Applikationsvorrichtung nach Anspruch 1 oder 2
**dadurch gekennzeichnet, daß**
die lichtstreuende Substanz Silikatkeramikpulver aufweist.

4. Applikationsvorrichtung nach einem der Ansprüche 1 bis 3
**dadurch gekennzeichnet, daß**
zumindest das Koppelmedium (6) von einer optisch transparenten Hülse (8) umgeben ist.

5. Applikationsvorrichtung nach einem der Ansprüche 1 bis 4
**dadurch gekennzeichnet, daß**
die Laserstrahlung unter einer von der Lichtleiterlängsachse abweichenden Richtung und/oder über eine schräg angeschliffene Faserstimfläche in den Faserkern (5) eingekoppelt wird.

## Claims

1. Application device for the treatment of biological tissue with laser radiation, having an optical waveguide (1) which guides the laser radiation and whose distal end has had the cladding (4) at least partially removed from it, so that the laser radiation emerges laterally from the fibre core (5), at least the positions of the distal optical-waveguide end from which the cladding (4) is removed being provided with a coupling medium (6) which transmits the radiation, which has a similar refractive index as the fibre core (5) and in which a light-scattering substance is incorporated, **characterised in that** the cladding (4) is removed in a plurality of annular regions (3), so that the lateral surface area of the exposed fibre core (5) per unit length increases exponentially in the distal direction.

2. Application device according to Claim 1, **characterised in that** the coupling medium (6) is a curable adhesive.

3. Application device according to Claim 1 or 2, **characterised in that** the light-scattering substance comprises silicate ceramic powder.

4. Application device according to one of Claims 1 to 3, **characterised in that** at least the coupling medium (6) is enclosed by an optically transparent sleeve (8).

5. Application device according to one of Claims 1 to 4, **characterised in that** the laser radiation is injected into the fibre core (5) in a direction which differs from the longitudinal axis of the optical waveguide and/or through an obliquely ground fibre end-face.

## Revendications

1. Dispositif d'application pour le traitement de tissus biologiques avec un rayonnement laser, comprenant un conducteur optique (1), guidant le rayonnement laser, dont l'extrémité distale est libérée au moins partiellement de la couche d'enveloppe (4), de telle sorte que le rayonnement laser sort par le côté du noyau de fibre (5), au moins les endroits libérés de la couche d'enveloppe (4) de l'extrémité distale du conducteur optique étant pourvus d'un agent de couplage (6) perméable pour le rayonnement laser, qui présente un indice de réfraction identique ou similaire au noyau de fibre (5) et dans lequel est logée une substance diffusant de la lumière, **caractérisé en ce que** la couche d'enveloppe (4) est enlevée dans plusieurs zones (3) de forme annulaire, de telle sorte que la couche d'enveloppe du noyau de fibre (5) mis à nu par unité de longueur augmente de façon exponentielle dans la direction distale.

2. Dispositif d'application selon la revendication 1, **caractérisé en ce que** l'agent de couplage (6) est une colle durcissable.

3. Dispositif d'application selon la revendication 1 ou 2, **caractérisé en ce que** la substance diffusant de la lumière comporte de la poudre céramique de silicate.

4. Dispositif d'application selon l'une quelconque des revendications 1 à 3, **caractérisé en ce que** au moins l'agent de couplage (6) est entouré d'une gaine (8) optiquement transparente.

5. Dispositif d'application selon l'une quelconque des revendications 1 à 4, **caractérisé en ce que** le rayonnement laser est intégré dans le noyau de fibre (5) dans une direction différente de l'axe longitudinal du conducteur optique et/ou sur une surface frontale de fibre meulée en biais.
